Europäisches Patentamt

European Patent Office

Office européen des brevets

(11) Veröffentlichungsnummer: **0 390 768**
**A1**

## (12) EUROPÄISCHE PATENTANMELDUNG

(21) Anmeldenummer: **90890060.8**

(22) Anmeldetag: **07.03.90**

(51) Int. Cl.5: **A61F 2/36**

(30) Priorität: **16.03.89 AT 602/89**

(43) Veröffentlichungstag der Anmeldung:
**03.10.90 Patentblatt 90/40**

(84) Benannte Vertragsstaaten:
**BE CH DE DK ES FR GB GR IT LI LU NL SE**

(71) Anmelder: **BÖHLER Gesellschaft m.b.H.**
**Nordwestbahnstrasse 12-14**
**A-1201 Wien(AT)**

(72) Erfinder: **Kaltenbrunner, Werner**
**Graschnitzstrasse 7A**
**A-8641 St. Marein(AT)**
Erfinder: **Kotz, Rainer, Prof., Dr.**
**Sonnenfelsgasse 11**
**A-1010 Wien(AT)**

(54) **Knochenimplantat.**

(57) Die Erfindung betrifft ein Knochenimplantat für Gelenksendoprothesen mit einem in eine Ausnehmung (1) eines Knochens (2) einsetzbaren, kragenlosen, kegelstumpfförmigen Schaft (3), von dem ein Gelenkteil (7) getragen ist, welcher Schaft (3) eine Mantelfläche (8) mit einer Mehrzahl von Vorsprüngen (9) aufweist. Erfindungsgemäß ist vorgesehen, daß die Vorsprünge (9) von lokalen, sich von der Mantelfläche (8) erhebenden Zähnen gebildet sind, deren distaler Bereich zum Abspanen des Knochens (2) während des Einsetzens bzw. zur Schaffung eines Raumes für die Vorsprünge (9) zum Einsetzen derselben in den Knochen (2) zumindest eine Schneid- bzw. Spankante bzw.-fläche (10) besitzt.

EP 0 390 768 A1

Fig. 1

## Knochenimplantat

Die Erfindung betrifft ein Knochenimplantat für Gelenksendoprothesen für den Bewegungs-, insbesondere Gehapparat des Menschen, mit einem in eine Ausnehmung eines Knochens einsetzbaren, von seinem distalen Ende zum proximalen Ende hin zumindest über einen Teilabschnitt zunehmende, Gesamtquerschnittsfläche aufweisenden, insbesondere kragenlosen, länglichen, vorzugsweise im wesentlichen kegelstumpfförmigen Schaft, von dem im Bereich seines proximalen Endes mittels eines einstückig oder gesondert gebildeten Zwischenstückes ein Gelenk bzw. Gelenkteil getragen ist, welcher insbesondere metallische Schaft im Knochen bevorzugt primär stabil verankerbar ist und eine bevorzugt bindemittelfrei mit dem Knochen in Kontakt bringbare Mantelfläche mit einer Mehrzahl von bevorzugterweise untereinander gleichartigen Vorsprüngen aufweist, die vorzugsweise im wesentlichen radial von der Hauptschaftachse weg nach außen hin weisen.

Es sind längliche, in Röhrenknochen einbringbare Gelenksimplantate bekannt, bei denen der Querschnitt vom proximalen zum distalen Endbereich hin abnimmt, um den Schaft an die Gegebenheiten des Knochens anzupassen bzw. die Materialabtragung bei der Herstellung der Ausnehmung zur Aufnahme des Implantates zu minimieren.

Zur Verankerung von Implantaten im Knochen sind einerseits der Einsatz von Bindemitteln, z.B. auf Kunststoffbasis, und andererseits bindemittelfrei arbeitende Techniken zur Verankerung bekanntgeworden, wobei bei Direktverankerung Probleme infolge Wärmeentwicklung und Gewebeunverträglichkeit des Bindemittels vermeidbar sind. Angestrebt wird allerdings , daß nach Einbringen des Schaftteiles nicht ein eine längere Zeitspanne benötigendes Einwachsen des Implantates abgewartet werden muß, sondern daß sofort beim Einbringen des Implantates eine stabile Verbindung zwischen Schaft und Knochen geschaffen wird.

Die Herstellung der Ausnehmung im Knochen für die Aufnahme des Schaftes des Implantates erfolgt im wesentlichen in der Weise, daß eine der Gestalt des Schaftes im wesentlichen entsprechende Raspel, Fräser, Räumwerkzeug od. dgl. in den Knochen gegen die Diaphyse hin vorgetrieben wird. Liegen die bekannten, verschiedenen meist gekrümmte Formen aufweisenden Implantatschafte nur an wenigen Stellen und über relativ kleine Bereiche an der Ausnehmungswandung an, so erfolgt an diesen Stellen die Einleitung der mechanischen Kräfte über Zonen relativ geringer Flächenausdehnung und damit eine hohe Druckbelastung. Solche Druckspitzen jedoch bewirken eine Störung des biologischen Gleichgewichtes im Knochen, auf welche dieser in der Weise reagiert, daß an diesen Stellen erhöhter Druckbeanspruchung ein Abbau der Knochensubstanz und damit eine Schwächung der Kortex und somit die Gefahr einer Lockerung des Sitzes des Schaftes eintritt.

Die Anordnung von längsverlaufenden Rippen an den Mantelflächen der Schäfte beseitigt dieses Problem nicht. Die Kanten dringen zwar in das Knochengewebe ein, verdrängen dieses jedoch seitlich, sodaß auch dadurch erhöhte Druckspannungen auftreten, welche auf Grund der Linienbelastung des Knochens diesen sprengen können. Ferner wird die Kontaktfläche zwischen dem Knochen und dem Schaft des Implantates durch diese Vorgangsweise nicht wesentlich vergrößert; es liegen in einem derartigen Fall nicht die Mantelfläche sondern im wesentlichen nur die längsverlaufenden Rippen am Knochen an bzw. dringen in diesen ein.

Aufgabe der vorliegenden Erfindung ist es, ein Knochenimplantat der eingangs genannten Art derart auszubilden, daß bei seiner einfachen Einbringung in die Ausnehmung des Knochens eine möglichst große Anlage der Mantelfläche des Schaftes an der Innenwand der Ausnehmung des Knochens ermöglicht wird und zusätzlich höchste Sicherheit gegen Axialverdrehung bei guter physiologischer Verträglichkeit und hoher Eignung für Langzeiteinsatz gegeben sind.

Erfindungsgemäß wird dieses Ziel bei einem Knochenimplantat der eingangs genannten Art dadurch erreicht, daß die Vorsprünge von sich lokal von der Mantelfläche erhebenden Zähnen gebildet sind, deren distaler Bereich zum Abspanen bzw. Abschaben des Knochens während des Einsetzens bzw. zur Schaffung eines Raumes für die Vorsprünge zum Einsetzen derselben in den Knochen zumindest eine Schneid-bzw. Spankante bzw.- fläche besitzt bzw. als Schneid- bzw. Spankante bzw. -fläche ausgebildet ist.

Das erfindungsgemäße Implantat erfordert nur die einfache Herstellung einer rotationssymmetrischen Ausnehmung im Knochen, womit bereits eine hohe Übereinstimmung zwischen der Form der Ausnehmungsinnenwand und der Mantelfläche des Schaftes erzielbar ist. Die an der Mantelfläche vorgesehenen Zähne bzw. Vorsprünge räumen die Knochensubstanz beim Einbringen des Implantates nach distal weg und verdrängen diese nicht seitlich. Dieses Wegräumen der Knochensubstanz beim Einbringen verhindert die Ausbildung von mechanischen Spannungen im Knochen. Gleichzeitig ist jedoch das Implantat gegen Verdrehen gesichert. Die relativ kurzen Zähne tragen den Knochen nur über kurze Bereich ab, d.h. über Bereiche, welche im wesentlichen der Längserstreckung der Zähne entsprechen, sodaß eine unnötige Schwächung des Knochens vermieden wird. Das Eindringen der Zähne in den Knochen verhindert aber nicht, daß die Mantelfläche des Schaftes des eingesetzten Implantates in guten Anlagenkontakt mit der Innenwandfläche der Ausnehmung gelangt.

Bevorzugt ist es, wenn der Schaft des Implantates einen zylindrischen und einen kegeligen Schaftteil besitzt, wobei der kegelige Schaftteil länger ist als der zylindrische Schaftteil und die Vorsprünge vorzugsweise im kegeligen Schaftteil des Implantates ausgebildet sind, wobei gegebenenfalls die Vorsprünge in zumindest zwei, insbesondere parallel zueinander verlaufenden, Umfangsreihen angeordnet sind. Dabei ist es sinnvoll, wenn die Umfangsreihen der Vorsprünge etwa im Bereich eines und zweier Drittel(s) der Länge des kegeligen Schaftes gelegen sind. Durch diese Anordnung der Zähne wird ausreichender Halt gegen eine Verdrehung geboten und es erfolgt eine knochenschonende Implantierung des Schaftes der Prothese.

Es zeigte sich, daß es vorteilhaft ist, wenn die Höhe der Vorsprünge über die Mantelfläche 0,2 bis 1 mm, vorzugsweise 0,3 bis 0,6 mm, beträgt. Zweckmäßig ist es dabei, wenn die über den Umfang des kegeligen Schaftteiles die in Abständen angeordneten Vorsprünge einen durch Zentriwinkel von 10 bis 35°, vorzugsweise 15 bis 30°, gegebenen Abstand besitzen. Diese Ausbildung, verbunden mit einer regelmäßigen Anordnung der Zähne über den Umfang des Schaftteiles, ermöglicht eine einfache Herstellung des Implantates, ein leichtes Einsetzen in die vorbereitete Ausnehmung im Knochen und bietet ausreichenden Halt.

Zur Aufnahme des von den Zähnen abgetragenen bzw. vor sich hergeschobenen Knochenmaterials ist es vorteilhaft, wenn vorzugsweise anschließend an den distalen Endbereich der Vorsprünge im kegeligen Schaftteil eine umlaufende Vertiefung bzw. Umfangsnut ausgebildet ist, oder wenn anschließend an den distalen Endbereich der Vorsprünge oder an die Umfangsnut der kegelige Schaftteil auf einen zylindrischen Schaftumfangsbereich abgetragen bzw. verringert ist, dessen Länge etwa der Längserstreckung der Vorsprünge entspricht. Damit werden Aufnahme- bzw. Ablagerungsräume für das Knochenmaterial geschaffen, die gleichzeitig ein Einwachsen des Knochens in Ausnehmungen der Prothese ermöglichen, womit der Widerstand gegen ein Herausziehen des Schaftes erhöht wird.

Wenn die Flächen zwischen den einzelnen Vorsprüngen eben ausgebildet sind bzw. diese ebenen Flächen in ihrem proximalen Endbereich mit kreissegmentförmigen, im wesentlichen halbkreisförmigen, auf gleichem radialen Niveau liegenden Flächenbereichen verlängert sind, wird eine Herstellung der Zähne mittels eines Winkelfräsers in einfacher Weise möglich, der in diesem Fall nur parallel zur Schafthauptachse verfahren werden muß, um die einzelnen Zähne auszubilden.

Weitere bevorzugte Ausführungsformen der Erfindung sind der folgenden Beschreibung, der Zeichnung und den Patentansprüchen zu entnehmen.

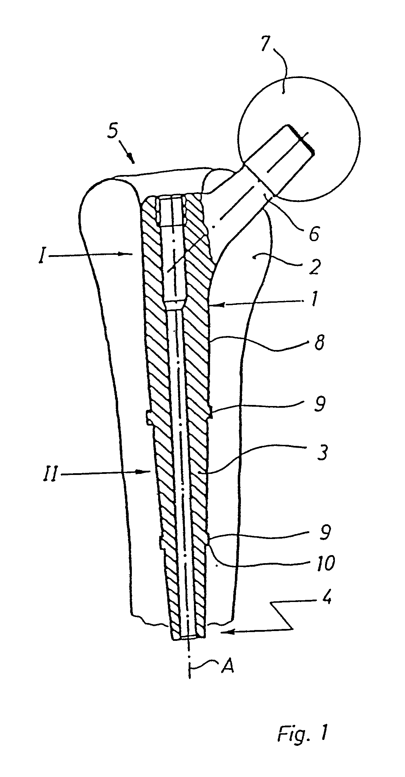

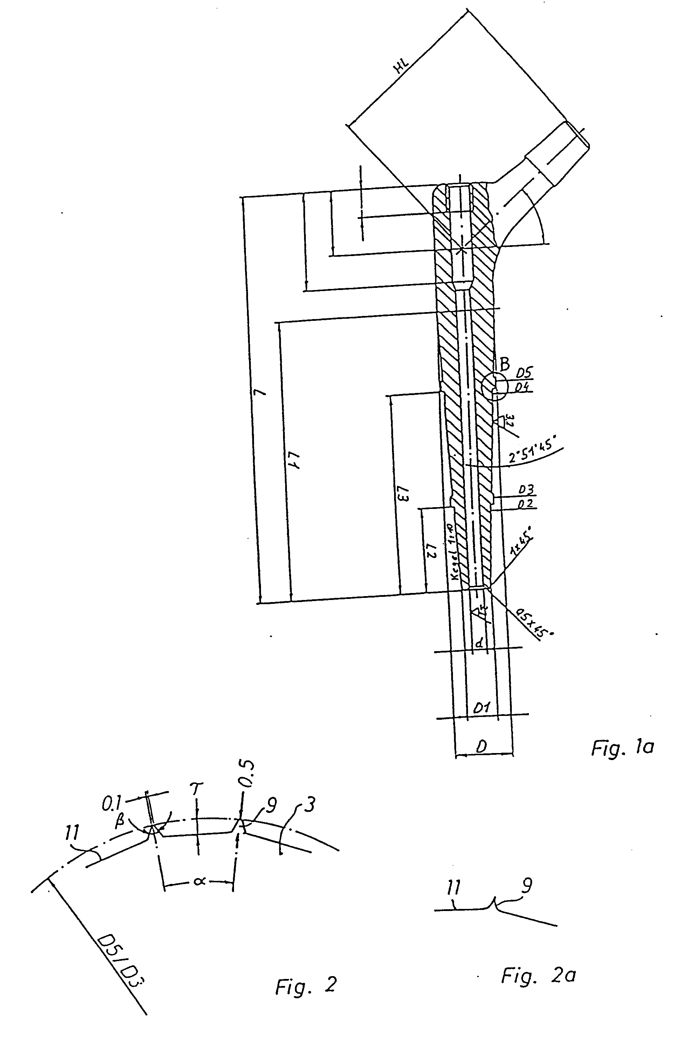

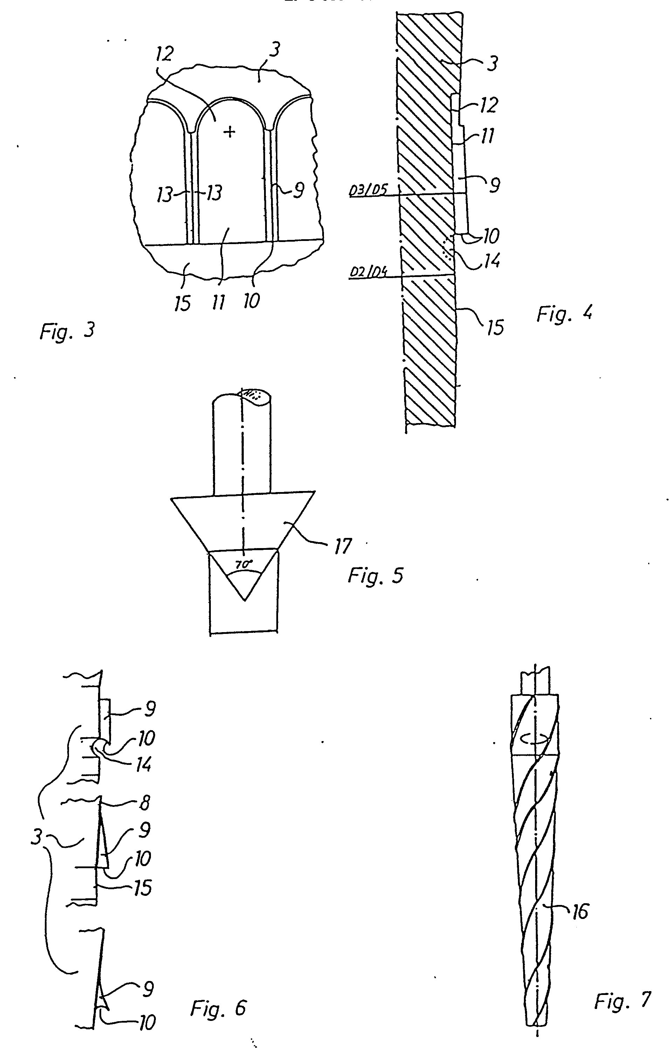

Im folgenden wird die Erfindung anhand der Zeichnung beispielsweise näher erläutert. Es zeigen Fig. 1 schematisch ein in einen Knochen eingestztes Implantat, Fig. 1a das Implantat in vergrößerter Ansicht, Fig. 2 und 2a Teilschnitte durch den Schaft eines Implantates in der Höhe einer Zahnreihe, Fig. 3 eine Draufsicht und Fig. 4 eine Längsansicht gemäß Detail B in Fig. 1a, Fig. 5 einen Winkelfräser, wie er zur Herstellung von Zähnen , z.B. gemäß Fig. 2, einsetzbar ist, Fig. 6 verschiedene Ausführungsformen von Zähnen und Fig. 7 ein Werkzeug zur Herstellung einer kegeligen Ausnehmung im Knochen.

Fig. 1 zeigt schematisch ein in einen Knochen 2 eingesetztes Implantat 1. Das Implantat besitzt einen zylindrischen Schaftteil I, an dem ein Zwischenstück 6 angeformt ist, an dessen Ende ein Gelenksteil 7, z.B. eine Gelenkskugel, angebracht bzw. aufgesetzt werden kann. Das Implantat wird durch eine im Knochen 2 ausgebildete Öffnung 5 in eine mit einem in Fig. 7 dargestellten Werkzeug 16 ausgebildete Ausnehmung eingebracht. An den zylindrischen Schaftteil I schließt ein sich kegelig verjüngender Schaftteil II an, der mit seiner Mantelfläche an der Innenwand der im Knochen 2 ausgebildeten Ausnehmung anliegt. Der kegelige Schaftteil II trägt Umfangsreihen von Vorsprüngen 9, welche beim Einsetzen des Schaftes 3 in den Knochen 2 diesen abspanen. Dazu sind die dem distalen Ende des Schaftes 3 zugekehrten Flächen bzw. Kanten der Vorsprünge als Schneidflächen bzw. Schneidkanten 10 ausgebildet.

Fig. 1a zeigt ein Implantat im vergrößerten Maßstab, wobei dieser Fig. die Abmessungen der einzelnen Bereiche des Implantates zu entnehmen sind, wie sie in der folgenden Tabelle angeführt sind. Diese Maßangaben sind die Abmessungen bevorzugter Ausführungsformen.

| D | d | L | HL | D1 | L1 | L2 | D2 | D3 | L3 | D4 | D5 | bei D3 | | | bei D5 | | |
|---|---|---|---|---|---|---|---|---|---|---|---|---|---|---|---|---|---|
| | | | | | | | | | | | | Z | α | T | Z | α | T |
| 18 | 5 | 135 | 53 | 9 | 90 | 27 | 11,1 | 12,5 | 58 | 14,2 | 15,6 | 12 | 30,0 | 0,64 | 15 | 24,0 | 0,61 |
| 19 | 6 | 135 | 54 | 10 | 90 | 27 | 12,1 | 13,5 | 58 | 15,2 | 16,6 | 13 | 27,69 | 0,63 | 16 | 22,5 | 0,60 |
| 20 | 6 | 145 | 54 | 10 | 100 | 27 | 12,1 | 13,5 | 68 | 16,2 | 17,6 | 13 | 27,69 | 0,63 | 17 | 21,18 | 0,59 |
| 21 | 6 | 145 | 55 | 11 | 100 | 27 | 13,1 | 14,5 | 68 | 17,2 | 18,6 | 14 | 25,71 | 0,62 | 18 | 20,0 | 0,59 |
| 22 | 7 | 145 | 56 | 12 | 100 | 27 | 14,1 | 15,5 | 68 | 18,2 | 19,6 | 15 | 24,0 | 0,61 | 19 | 18,95 | 0,58 |
| 23 | 8,5 | 150 | 56 | 12,5 | 105 | 33 | 15,2 | 16,6 | 74 | 19,3 | 20,7 | 16 | 22,5 | 0,60 | 20 | 18,0 | 0,58 |
| 24 | 8,5 | 160 | 57 | 12,5 | 115 | 33 | 15,2 | 16,6 | 74 | 19,3 | 20,7 | 16 | 22,5 | 0,60 | 20 | 18,0 | 0,58 |
| 25 | 8,5 | 165 | 58 | 13 | 120 | 38 | 16,2 | 17,6 | 80 | 20,4 | 21,8 | 17 | 21,18 | 0,59 | 21 | 17,14 | 0,58 |
| 26 | 9 | 165 | 59 | 14 | 120 | 38 | 17,2 | 18,6 | 80 | 21,4 | 22,8 | 18 | 20,0 | 0,59 | 22 | 16,36 | 0,57 |
| 27 | 9 | 165 | 60 | 15 | 120 | 38 | 18,2 | 19,6 | 80 | 22,4 | 23,8 | 19 | 18,95 | 0,58 | 23 | 15,65 | 0,57 |

Fig. 2 zeigt einen Teilschnitt senkrecht zur Schafthauptachse A durch zwei Vorsprünge 9. Man erkennt, daß die Fläche 11 zwischen den Vorsprüngen eben ausgebildet wurde, z.B. unter Zuhilfenahme eines in Fig. 5 dargestellten Winkelfräsers 17. Der Spitzenwinkel des Winkelfräsers 17 bestimmt den Winkel der Seitenflächen der Vorsprünge 9, welchen diese mit der Fläche 11 einschließen und somit auch den Winkel β zwischen den Seitenflächen der im wesentlichen dreieckförmigen Querschnitt besitzenden Vorsprünge 9. Man erkennt aus Fig. 2, daß an der Spitze der Vorsprünge 9 eine Rundung von 0,5 mm ausgebildet sein kann. Ferner ist in Fig. 2 der Verzahnungswinkel α angegeben, welcher durch die Zähnezahl Z bestimmt, wenn über den Umfang des Schaftes 3 eine regelmäßige Anordnung der Vorsprünge 9 erfolgt. In Fig. 2a ist ein Schnitt durch einen Vorsprung 9 mit konkaven Seitenflächen dargestellt.

Fig. 3 zeigt eine Draufsicht auf einen Bereich des Schaftes 3 mit Zähnen 9. Man erkennt die zwischen den Zähnen 9 verlaufenden, im wesentlichen ebenen Flächen 11, sowie an die Flächen 11 in Richtung des proximalen Endes des Schaftes 3 anschließenden halbkreisförmigen Flächen 12, welche durch den Einsatz des Winkelfräsers 17 ausgebildet werden. Diese Flächen sind von Bedeutung, da sie einen Durchfluß von Körperflüssigkeiten zulassen können und andererseits ein Einwachsen des Knochens zulassen und einem Verdrehen bzw. einem Eindrücken des Schaftes 3 entgegenwirken. Am distalen Ende der Vorsprünge 9 liegen die Zahnflächen 10. Die Vorsprünge gehen vorteilhafterweise in einen zylindrischen abgedrehten Bereich 15 des kegeligen Schaftteiles II über, um durch die Querschnittsreduktion des kegeligen Schaftteiles II im Bereich 15 eine Ablagerungsmöglichkeit für abgeschabtes Knochenmaterial zu bilden.

Diese Ausbildung ist in Fig. 4 in Schnittdarstellung näher erläutert. Man erkennt die Verlängerungsfläche 12, die Fläche 11 zwischen den Zähnen 9, die Räum- bzw. Schneidfläche bzw. -kante 10 und die anschließende zylindrische Umfangsfläche 15. Anstelle dieser zylindrischen Umfangsfläche 15 oder zusätzlich kann eine Umfangsnut 14 anschließend an die Räumflächen 10 ausgebildet sein.

Fig. 6 zeigt eine Anordnung von verschiedenen Zähnen 9. Es ist verständlich, daß auch die Form der Räumzähne variierbar ist. Im oberen Bereich der Fig. 6 erkennt man einen Zahn 9 mit einer Räumfläche 10, die einen Winkel mit der Mantelfläche 8 des kegeligen Schaftteiles II einschließt, der beträchtlich kleiner als 90° ist. An die Räumfläche 10 schließt eine Umfangsnut 14 an. Dieser Zahn weist eine im wesentlichen zur Schafthauptachse A parallele Rückenkante auf und ist an seinem proximalen Ende abgesetzt.

In Fig. 6 Mitte ist ein Räumzahn 9 dargestellt, an dessen Räumfläche 10 ein abgedrehter Abschnitt 15 des kegeligen Schaftteiles II anschließt. Der Rücken des Räumzahnes 9 verläuft geneigt zur Schafthauptachse A. Die Räumfläche 10 steht senkrecht zur Mantelfläche 8.

In Fig. 6 unten ist ein Zahn 9 mit konkaver Räumfläche 10 und konkaver Rückenkante dargestellt.

Die Herstellung der Zähne kann auf verschiedene Art erfolgen. Die vorteilhafteste und genaueste Art der Herstellung ist die Herstellung mit einem Fräskopf, wie er in Fig. 5 dargestellt ist. Es sind jedoch auch andere Herstellungsarten, welche gegebenenfalls einen geringeren Zentriwinkel bedingen, z.B. Rändeln, Meißeln, Schlagen od. dgl., möglich, soferne man darauf achtet, daß bei diesen Vorgängen die distalen Flächen 10 der Zähne 9 in gewünschter Weise entsprechend scharf zum Abspanen bzw. Abräumen von Knochenmaterial ausgebildet werden. Vorteilhaft ist es deshalb, wenn die Höhe der Vorsprünge zum distalen Ende des Schaftes hin gleichbleibt oder zunimmt. Eine Abnahme in diese Richtung würde unerwünschte mechanische Spannungen im Knochen durch Verdrängung von Knochenmaterial seitlich zur Richtung des Einschiebens des Implantates bewirken. Schneidflächen 10, die mit der Mantelfläche 8 einen Winkel einschließen , der kleiner als 90° ist, sind vorteilhaft, da dadurch eine definierte Ablagerung des abgeschabten Knochenmaterials in den Nuten 14 erfolgt.

Vorteilhafterweise erfolgt vor Ausbildung der Zähne 9 , Flächen 11,12, Umfangsnuten 14, zylindrischer Bereiche 15 ein Strahlen, z.B. Sandstrahlen oder Kugelstrahlen des Schaftes 3, um den unmittelbaren Halt des Implantates 1 zu erhöhen. Die Ausbildung der Zähne wird erst nach dem Sandstrahlen vorgenommen, so daß diese blank vorliegen und ihre Schneidfähigkeit durch ein Strahlen nicht verlorengeht. Werden die Zähne vor dem Sandstrahlen ausgebildet, werden sie während des Sandstrahlens abgedeckt.

Vorteilhafterweise ist vorgesehen, daß der Schaft 3 einen geraden bzw. rotationssymmetrischen sich kegelig verjüngenden distalen Schaftteil II und einen anschließenden zylindrischen, das Zwischenstück 6 tragenden, proximalen Schaftteil I besitzt, wobei das Längenverhältnis des zylindrischen I zum kegeligen II Schaftteil im Bereich von 0,2 bis 0,4, vorzugsweise von 0,25 bis 0,35, liegt bzw. daß das Verhältnis der Differenzen der Durchmesser ( D-d), gemessen an den jeweiligen Enden des kegeligen Schaftteiles II zu der Länge des kegeligen Schaftteiles II im Bereich von 1:8 bis 1:12, vorzugsweise von 1:9 bis 1:11, gelegen ist. Dabei ist es zweckmäßig, wenn die Länge der Vorsprünge 9 in Richtung der Hauptschaftachse A gemessen etwa 1/40 bis 1/20 der Länge des kegeligen Schaftteiles II beträgt. Zweckmäßig ist es ferner, wenn der Kantenwinkel zwischen den Seitenflächen 13 der Vorsprünge 9 60° bis 80°, insbesondere etwa 70°, beträgt.

Es ist verständlich, daß auch eine unregelmäßige Verteilung der Zähne über den Schaft 3 bzw. kegeligen Schaftteil II möglich ist. Allzuviele Zähne sollen jedoch durch die dadurch erfolgende Belastung des Knochens nicht vorgesehen werden. Kurze und nicht hohe Zähne sind vorzuziehen, da die Lage des Implantates in diesem Fall durch die Anlage der Mantelfläche an die Trennwand der Ausnehmung und nicht durch die Anlage der Zähne an den Knochen bestimmt wird.

Als Material für das Implantat kommen vor allem Co-Basis-Legierungen und Ti-Legierungen mit Al, Fe, V, Mo oder dgl. in Frage.

Wenn der Schaft 3 eine Längsbohrung besitzt, können die Körperflüssigkeiten beim Einschlagen des Implantates durch diese entweichen.

## Ansprüche

1. Knochenimplantat für Gelenksendoprothesen für den Bewegungs-, insbesondere Gehapparat des Menschen, mit einem in eine Ausnehmung (1) eines Knochens (2) einsetzbaren, von seinem distalen Ende (4) zum proximalen Ende hin zumindest über einen Teilabschnitt zunehmende Gesamtquerschnittsfläche aufweisenden, insbesondere kragenlosen, länglichen, vorzugsweise im wesentlichen kegelstumpfförmigen Schaft (3), von dem im Bereich seines proximalen Endes (5) mittels eines einstückig oder gesondert gebildeten Zwischenstückes (6) ein Gelenk bzw. Gelenkteil (7) getragen ist, welcher Schaft (3) eine bevorzugterweise bindemittelfrei mit dem Knochen (2) in Kontakt bringbare Mantelfläche (8) mit einer Mehrzahl von vorteilhafterweise untereinander gleichartigen, nach außen gerichteten Vorsprüngen (9) aufweist, dadurch gekennzeichnet, daß die Vorsprünge (9) von lokalen, sich von der Mantelfläche (8) erhebenden Zähnen gebildet sind, deren distaler Bereich zum Abspanen bzw. Abschaben des Knochens (2) während des Einsetzens bzw. zur Schaffung eines Raumes für die Vorsprünge (9) zum Einsetzen derselben in den Knochen (2) zumindest eine Schneid- bzw. Spankante bzw. -fläche (10) besitzt bzw. als Schneid- bzw. Spankante bzw. -fläche ausgebildet ist.

2. Implantat nach Anspruch 1, dadurch gekennzeichnet, daß der Schaft (3) des Implantates (1) einen zylindrischen (I) und einen kegeligen (II) Schaftteil besitzt, wobei der kegelige Schaftteil (II) länger ist als der zylindrische Schaftteil (I).

3. Implantat nach Anspruch 1 oder 2, dadurch gekennzeichnet, daß die Vorsprünge (9) in zumindest zwei, insbesondere parallel zueinander verlaufenden, Umfangsreihen angeordnet sind.

4. Implantat nach einem der Ansprüche 1 bis 3, dadurch gekennzeichnet, daß die Vorsprünge (9) eine im wesentlichen in Richtung auf das distale Ende des Implantates (1) weisende gegebenenfalls konkave Spankante bzw. -fläche (10) besitzen, die einen 90° nicht übersteigenden Winkel, vorzugsweise einen zwischen 45 und 90° gelegenen Winkel mit der Mantelfläche (8) einschließt.

5. Implantat nach einem der Ansprüche 1 bis 4, dadurch gekennzeichnet, daß die Höhe der Vorsprünge (9) in Richtung zum distalen Ende des Schaftes (3) hin gleichbleibt oder zunimmt.

6. Implantat nach einem der Ansprüche 1 bis 5, dadurch gekennzeichnet, daß die Vorsprünge (9) durch Rändeln, Fräsen, Meißeln od. dgl. am Schaft (3) ausgebildet sind.

7. Implantat nach einem der Ansprüche 1 bis 6, dadurch gekennzeichnet, daß der Schaft (3) des Implantates (1) mit Ausnahme der Vorsprünge (9) zumindest teilweise sandgestrahlt ist.

8. Implantat nach einem der Ansprüche 1 bis 7, dadurch gekennzeichnet, daß der Schaft (3) einen sich kegelig verjüngenden (II) distalen und einen anschließenden zylindrischen, das Zwischenstück (6) tragenden, proximalen Schaftteil (I) besitzt und das Längenverhältnis des zylindrischen (I) zum kegeligen (II) Schaftteil im Bereich von 0,2 bis 0,4, vorzugsweise von 0,25 bis 0,35, liegt.

9. Implantat nach einem der Ansprüche 1 bis 8, dadurch gekennzeichnet, daß die Umfangsreihen der Vorsprünge (9) etwa im Bereich eines und zweier Drittel(s) der Länge des kegeligen Schaftteiles (II) gelegen sind.

10. Implantat nach einem der Ansprüche 1 bis 9, dadurch gekennzeichnet, daß das Verhältnis der Differenzen der Durchmesser ( D.d), gemessen an den Enden des kegeligen Schaftteiles (II) zu der Länge des kegeligen Schaftteiles (II) im bereich von 1:8 bis 1:12, vorzugsweise von 1:9 bis 1:11, insbesondere bei 1:10 gelegen ist.

11. Implantat nach einem der Ansprüche 1 bis 10, dadurch gekennzeichnet, daß die Länge der Vorsprünge (9) in Richtung der Hauptschaftachse (A) gemessen etwa 1/40 bis 1/20 der Länge des kegeligen Schaftteiles (II) beträgt.

12. Implantat nach einem der Ansprüche 1 bis 11, dadurch gekennzeichnet, daß die Höhe der Vorsprünge (9) über die über die Mantelfläche (8) 0,2 bis 1 mm, vorzugsweise 0,3 bis 0,6 mm, beträgt.

13. Implantat nach einem der Ansprüche 1 bis 12, dadurch gekennzeichnet, daß die über den Umfang des kegeligen Schaftteiles (II) in Abständen angeordneten Vorsprünge (9) einen durch einen Zentriwinkel ($\alpha$) von 10 bis 35°, vorzugsweise 15 bis 30°, gegebenen Abstand besitzen.

14. Implantat nach einem der Ansprüche 1 bis 13, dadurch gekennzeichnet, daß die Vorsprünge (9) regelmäßig über den Umfang und gegebenenfalls über die Länge des kegeligen Schaftteiles (II) verteilt sind.

15. Implantat nach einem der Ansprüche 1 bis 14, dadurch gekennzeichnet, daß der Kantenwinkel ($\beta$) zwischen den Seitenflächen (13) der Vorsprünge (9) 60 bis 80°, insbesondere etwa 70°, beträgt.

16. Implantat nach einem der Ansprüche 1 bis 15, dadurch gekennzeichnet, daß vorzugsweise anschließend an den distalen Endbereich der Vorsprünge (9) im kegeligen Schaftteil (II) eine umlaufende Vertiefung bzw. Umfangsnut (14) ausgebildet ist.

17. Implantat nach einem der Ansprüche 1 bis 16, dadurch gekennzeichnet, daß anschließend an den distalen Endbereich der Vorsprünge (9) oder an die Umfangsnut (14) der kegelige Schaftteil (II) auf einen zylindrischen Schaftumfangsbereich (15) abgetragen bzw. verringert ist, dessen Länge etwa der Längserstreckung der Vorsprünge (9) entspricht.

18. Implantat nach einem der Ansprüche 1 bis 17, dadurch gekennzeichnet, daß die Flächen (11) zwischen den einzelnen Vorsprüngen (9) eben ausgebildet sind.

19. Implantat nach Anspruch 18, dadurch gekennzeichnet, daß die Flächen (11) ihrem proximalen Endbereich mit kreissegmentförmigen, im wesentlichen halbkreisförmigen, auf gleichem radialen Niveau liegenden Flächenbereichen (12) verlängert sind.

20. Implantat nach einem der Ansprüche 1 bis 19, dadurch gekennzeichnet, daß über die Längserstrekkung der Mantelfläche (8) des kegeligen Schaftabschnittes (II) aufeinanderfolgend , gegebenenfalls zueinander versetzt, eine Anzahl, vorzugsweise zumindest zwei, von Zähnen () angeordnet ist, deren Längserstreckung etwa 1/20 bis 1/40 der Länge des kegeligen Schaftteiles (II) entspricht.

21. Implantat nach einem der Ansprüche 1 bis 20, dadurch gekennzeichnet, daß die Abstände der Vorsprünge (9) in Längsrichtung des Schaftes (3) zumindest das 4-Fache, vorzugsweise das 5-Fache, insbesondere das 6-Fache, der Länge der Vorsprünge (9) betragen.

22. Implantat nach einem der Ansprüche 1 bis 21, dadurch gekennzeichnet, daß die Vorsprünge (9) im kegeligen Schaftteil (II) und gegebenenfalls auch im zylindrischen Schaftteil (I) ausgebildet sind.

23. Implantat nach einem der Ansprüche 1 bis 22, dadurch gekennzeichnet, daß die Rücken(kanten) der Vorsprünge (9) parallel zur Schafthauptachse verlaufen.

24. Implantat nach einem der Ansprüche 1 bis 23, dadurch gekennzeichnet, daß im Schaft (3) eine durchgehende, vorzugsweise zentrisch verlaufende Längsbohrung vorgesehen ist.

Fig. 1

Fig. 1a

Fig. 2

Fig. 2a

Fig. 3

Fig. 4

Fig. 5

Fig. 6

Fig. 7

# EUROPÄISCHER RECHERCHENBERICHT

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | US-A-3 067 740 (E.J. HABOUSH) * Figuren 1, 2A-C; Spalte 2, Zeilen 37-51 * | 1 | A 61 F 2/36 |
| A | --- | 2,14,17 | |
| X | US-A-4 714 470 (J.D. WEBB et al.) * Spalte 3, Zeilen 46-68; Figuren 1,8 * | 1 | |
| A | --- | 3 | |
| X | DE-U-8 127 991 (DR. MED. H. KUENNE) * Figur; Seite 2, Zeile 8 - Seite 3, Zeile 7 * | 1 | |
| A | --- | 6,12 | |
| A | EP-A-0 093 378 (W. LINK GMBH & CO.) * Seite 12, Zeilen 11-17; Figuren 1,2 * --- | 1 | |
| A | DE-B-2 839 092 (H. REIMER) * Anspruch 1; Figuren 1-3 * --- | 1,5,24 | |
| A | EP-A-0 209 516 (BOEHLER GMBH) * Anspruch 1; Figuren 1,2 * ----- | 1,24 | RECHERCHIERTE SACHGEBIETE (Int. Cl.5) A 61 F |

Der vorliegende Recherchenbericht wurde für alle Patentansprüche erstellt

| Recherchenort | Abschlußdatum der Recherche | Prüfer |
|---|---|---|
| BERLIN | 26-06-1990 | KANAL P K |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
........................................................................
& : Mitglied der gleichen Patentfamilie, übereinstimmendes Dokument